(19) Europäisches Patentamt
European Patent Office
Office européen des brevets

(11) Veröffentlichungsnummer : **0 137 254**
**B1**

(12) # EUROPÄISCHE PATENTSCHRIFT

(45) Veröffentlichungstag der Patentschrift : **03.12.86**

(21) Anmeldenummer : **84110003.5**

(22) Anmeldetag : **22.08.84**

(51) Int. Cl.[4] : **C 07 C 69/63**, C 07 C 67/29, C 07 C 67/343// C07C67/00, C07C69/16, C07C69/732

(54) **Halogen enthaltende Acyloxy-methyl-butene.**

(30) Priorität : **31.08.83 DE 3331399**

(43) Veröffentlichungstag der Anmeldung : **17.04.85 Patentblatt 85/16**

(45) Bekanntmachung des Hinweises auf die Patenterteilung : **03.12.86 Patentblatt 86/49**

(84) Benannte Vertragsstaaten : **CH DE FR GB IT LI**

(56) Entgegenhaltungen : **EP-A- 0 089 586**

(73) Patentinhaber : **BASF Aktiengesellschaft**
**Carl-Bosch-Strasse 38**
**D-6700 Ludwigshafen (DE)**

(72) Erfinder : **Fischer, Rolf, Dr.**
**Bergstrasse 98**
**D-6900 Heidelberg (DE)**
Erfinder : **Weitz, Hans-Martin, Dr.**
**Auf dem Koeppel 40**
**D-6702 Bad Duerkheim (DE)**
Erfinder : **Schmieder, Klaus, Dr.**
**Sonnenstrasse 12 b**
**D-6710 Frankenthal (DE)**
Erfinder : **Paust, Joachim, Dr,**
**Ringstrasse 3**
**D-6708 Neuhofen (DE)**

EP 0 137 254 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Diese Erfindung betrifft neue Halogen enthaltende Acyloxy-methyl-butene, ihre Herstellung und Verwendung.

Die neuen Halogen enthaltenden Acyloxy-methyl-butene haben die Formel

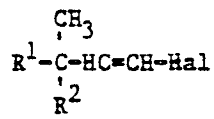

(I)

in der $R^1$ den

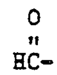

Rest oder zusammen mit $R^2$ einen

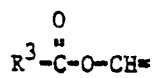

Rest, $R^2$ einen

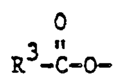

Rest oder zusammen mit $R^1$ einen

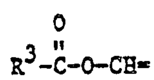

Rest, Hal ein Chlor- oder Bromatom und $R^3$ ein Wasserstoffatom oder einen Alkylrest mit 1 bis 16 C-Atomen bedeuten.

Verbindungen der genannten Art sind 1-Acyloxy-2-methyl-4-halogen-1,3-butadiene der Formel

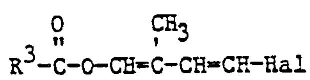

(II)

oder 2-Methyl-2-acyloxy-4-halogen-3-butenale der Formel

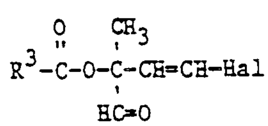

(III)

in denen Hal und $R^3$ die oben genannten Bedeutungen haben.

In den neuen Halogen enthaltenden Acyloxymethylbutenen bedeutet der Rest $R^3$ ein Wasserstoffatom oder einen Alkylrest mit 1 bis 16, vorzugsweise 1 bis 4 C-Atomen, wie einen Methyl-, Ethyl-, n-Propyl-, i-Propyl-, n-Butyl-, i-Butyl-, tert.-Butyl-, n-Pentyl-, Palmityl- oder Stearylrest.

Als 1-Acyloxy-2-methyl-4-halogen-1,3-butadienne der Formel II seien beispielsweise die folgenden Verbindungen genannt : 1-Acetoxy-2-methyl-4-chlor-1,3-butadien, 1-Acetoxy-2-methyl-4-brom-1,3-butadien, 1-Propionyloxy-2-methyl-4-chlor-1,3-butadien und 1-Palmityloxy-2-methyl-4-chlor-1,3-butadien.

Als 2-Methyl-2-acyloxy-4-halogen-3-butenale der Formel III seien beispielsweise die folgenden Verbindungen genannt : 2-Methyl-2-acetoxy-4-chlor-3-butenal, 2-Methyl-2-acetoxy-4-brom-3-butenal, 2-Methyl-2-propionyloxy-4-chlor-3-butenal und 2-Methyl-2-palmityloxy-4-chlor-3-butenal.

Die neuen 1-Acyloxy-2-methyl-4-halogen-1,3-butadiene der Formel II lassen sich durch Umsetzung von 2-Methyl-4-halogen-2-butenalen mit Acetylierungsmitteln, wie Acetanhydrid in Gegenwart von Carbonsäuren und Alkali- oder Erdalkalicarboxylaten herstellen. 2-Methyl-4-halogen-2-butenale, wie 2-Methyl-4-chlor-2-butenal, sind beispielsweise durch Chlorierung von 3-Methyl-3,4-epoxy-2-buten (Isoprenepoxid) (Journ. Org. Chem. *41*, Seiten 1648-1650 (1976)) oder trans-2-Methyl-2-butenal (Tiglinaldehyd) (Tetrahedron Letters *20*, Seiten 1719-1720 (1973)) mit Kupfer(II)chlorid zugänglich.

Die neuen 2-Methyl-2-acyloxy-4-halogen-3-butenale der Formel III lassen sich z. B. dadurch herstellen, daß man 1-Acyloxy-2-methyl-4-halogen-1,3-butadiene der Formel II mit Sauerstoff oder Sauerstoff abgebenden Mitteln umsetzt. Dieses Verfahren läßt sich für den Fall der Synthese von 2-Methyl-2-acetoxy-4-chlor-3-butenal mit Hilfe von Peressigsäure in Eisessig als Lösungsmittel durch die folgenden Formeln anschaulich machen :

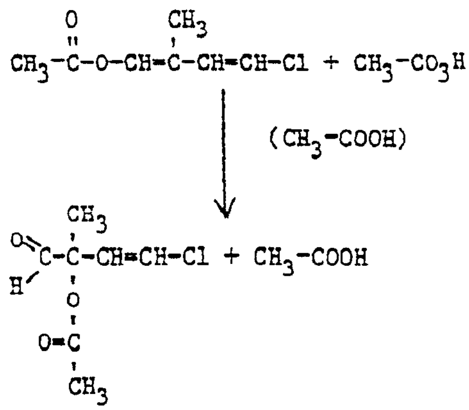

Als Sauerstoff abgebende Verbindungen sind z. B. solche geeignet, die für die Epoxidierung von Olefinen benutzt werden, wie Wasserstoffperoxid, Persäuren, Perameisensäure, Peressigsäure, Perpropionsäure, Perbenzoesäure, Per-n-buttersäure, Per-iso-buttersäure und organische Hydroperoxide wie tert.-Butylhydroperoxid oder Cumolhydroperoxid. Verbindungen dieser Art werden z. B. in Ullmanns Encyclopädie der Technischen Chemie, 4. Auflage, Band 10, Seiten 563 bis 567 genannt. Der Sauerstoff und die Sauerstoff abgebenden Verbindungen können auch in Gegenwart von Katalysatoren verwendet werden. Sauerstoff kann in Form von reinem Sauerstoff oder im Gemisch mit anderen Gasen, wie Stickstoff z. B. in Form von Luft oder mit anderen inerten Gasen wie Kohlendioxid verwendet werden.

Das Verfahren zur Herstellung der Verbindungen der Formel III wird z. B. so durchgeführt, daß man pro Mol 1,3-Dien der Formel II 0,5 bis 10 Mole, insbesondere 1 bis 1,5 Mole Sauerstoff oder Sauerstoff abgebende Verbindung anwendet. Man arbeitet zweckmäßig bei Temperaturen zwischen 0 und 200 °C, insbesondere bei 20 bis 120 °C und wendet z. B. Sauerstoffdrucke von 1 bis 100, insbesondere 1 bis 20 bar an. Das Verfahren kann chargenweise oder kontinuierlich, drucklos oder unter Druck durchgeführt werden. Unumgesetzte 1,3-Diene der Formel II können gegebenenfals nach der Umsetzung destillativ von den entstandenen 2-Methyl-2-acyloxy-4-halogen-3-butenalen der Formel III abgetrennt und erneut für die erfindungsgemäße Umsetzung verwendet werden.

Gewöhnlich arbeitet man entweder in einem Überschuß einer Carbonsäure wie Essigsäure oder Propionsäure als Lösungsmittel oder in Gegenwart von unter den Reaktionsbedingungen inerten Lösungsmitteln. Als Lösungsmittel dieser Art kommen z. B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole, Ether, wie Diethylether, Tetrahydrofuran oder Dioxan in Betracht. Pro Mol Ausgangsverbindung der Formel II verwendet man zweckmäßigerweise 1 bis 80 Mole, insbesondere 2 bis 60 Mole, des unter den Reaktionsbedingungen inerten Lösungsmittels.

Die Umsetzung kann man bei diskontinuierlicher Arbeitsweise z. B. wie folgt vornehmen : Einer Lösung des 1,3-Diens der Formel II in dem jeweiligen Lösungsmittel werden bei der Reaktionstemperatur und dem Reaktionsdruck Sauerstoff oder die Sauerstoff abgebende Verbindung zugeführt. Nach beendeter Zugabe wird gegebenenfalls noch nachgerührt. Durch das auf Raumtemperatur abgekühlte Reaktionsgemisch wird gegebenenfalls Stickstoff geleitet. Anschließend wird nach dem Abdestillieren des Lösungsmittels fraktioniert destilliert. Dabei werden gegebenenfalls nicht umgesetzte Ausgangsverbindungen von den gewünschten Aldehyden abgetrennt.

Es ist auch möglich, als Ausgangsverbindungen nicht reine 1-Acyloxy-2-methyl-4-halogen-1,3-butadiene der Formel II, sondern Gemische aus II mit 2-Methyl-1,4-diacyloxy-1,3-butadienen zu verwenden. Nach der Umsetzung dieser Gemische mit Sauerstoff abgebenden Verbindungen erhält man Gemische aus 2-Methyl-2-acyloxy-4-halogen-3-butenalen III und 2-Methyl-4,4-diacyloxy-2-butenalen, die entweder destillativ getrennt oder als solche verwendet werden können. 2-Methyl-4,4-diacyloxy-2-butenale stellen ebenfalls geeignete Verbindungen für die Synthese von Terpenen dar (DE-OS 31 25 891).

Die nach dem erfindungsgemäßen Verfahren mit hoher Selektivität zugänglichen neuen 2-Methyl-2-acyloxy-4-halogen-3-butenale der Formel III stellen wertvolle Zwischenprodukte für die Herstellung von Terpenen, wie Retinal, β-Carotin (DE-OS 23 57 810) und Apocarotinoiden dar. Sie lassen sich mit Phosphoniumyliden durch Wittigreaktion zu Verbindungen der Formel

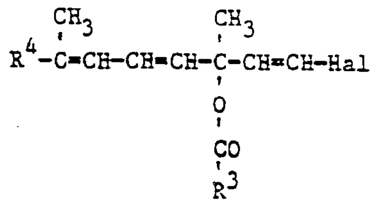

umsetzen. In der Formel IV haben Hal und $R^3$ die obengenannte Bedeutung und $R^4$ bedeutet einen $R^5OOC$-Rest, eine Formylgruppe oder eine acetalisierte Formylgruppe, wobei $R^5$ für einen niedermolekularen Alkylrest, wie einen Alkylrest mit 1 bis 6 C-Atomen, steht.

Verbindungen der Formel IV ergeben nach Allylumlagerung und Hydrolyse als Terpenbausteine verwendbare Verbindungen.

Die Bildung der Verbindungen der Formel III ist überraschend, da man in Analogie zur Bildung von 2-Alkyl-4,4-diacyloxy-2-butenalen durch Umsetzung von 2-Alkyl-1,4-diacyloxy-1,3-butadienen mit Sauerstoff oder Sauerstoff abgebenden Verbindungen in Carbonsäuren (DE-OS 31 25 891) die Entstehung von 2-Methyl-4-halogen-4-acyloxy-2-butenalen und nicht von 2-Methyl-2-acyloxy-4-halogen-3-butenalen hätte erwarten müssen. Aus der EP-PS 5452 is außerdem bekannt, daß bei der Umsetzung von 2-Alkyl-1,4-diacyloxy-1,3-butadienen mit Carbonsäuren und Sauerstoff in Gegenwart von Palladium oder Platin enthaltenden Katalysatoren 2-Alkyl-1,1,4,4-tetraacyloxy-2-butene gebildet werden.

Beispiel 1

Herstellung von 1-Acetoxy-2-methyl-4-chlor-1,3-butadien (s. Formel II mit $R^3 = CH_3$ ; Hal = Cl)

In einem 2 Liter-Dreihalskolben mit Gaseinleitungsrohr, Rückflußkühler, Magnetrührer und Innentehermometer wurde ein Gemisch aus 449 g Acetanhydrid, 53 g Essigsäure und 47 g Kaliumacetat auf 110 °C erwärmt. In den Gasraum über der Lösung wurde Stickstoff geleitet. Nach Zugabe von 25,2 g 2-Methyl-4-chlor-2-butenal innerhalb von 2 Minuten wurde das Reaktionsgemisch unter Stickstoff 16 Stunden bei 110 ± 1 °C gerührt. Nach dem Abkühlen auf 20 bis 30 °C wurde mit 1 200 ml Wasser versetzt und eine Stunde bei Raumtemperatur gerührt. Die Phasen wurden im Scheidetrichter getrennt. Die Wasserphase wurde sechsmal mit je 70 ml Petroleumbenzin (Siedebereich 40 bis 60 °C) extrahiert. Diese Extrakte wurden mit der organischen Phase vereinigt und über Natriumsulfat getrocknet. Nach dem Abfiltrieren des Trockenmittels wurde das Lösungsmittel am Rotationsverdampfer (30 bis 40 °C/20 mbar) abgezogen. Durch fraktionierte Destillation des Rückstandes (39,6 g) wurden 12,5 g 2-Methyl-1,4-diacetoxy-1,3-butadien vom Siedepunkt 55 bis 63 °C/0,2 mbar, $n_D^{20} = 1.485\,5$ (32 % d. Th., bezogen auf eingesetztes 2-Methyl-4-chlor-2-butenal) und 15,5 g 1-Acetoxy-2-methyl-4-chlor-1,3-butadien vom Siedepunkt 41 bis 42 °C/0,2 mbar, $n_D^{20} = 1.508\,4$ (46 % d. Th., bezogen auf eingesetztes 2-Methyl-4-chlor-2-butenal) erhalten.

$^1$H-NMR-Spektrum von 1-Acetoxy-2-methyl-4-chlor-1,3-butadien (Lösungsmittel $CDCl_3$, TMS als innerer Standard : $\delta$ = 1.80 (d. 3H), 2.19 (s, 3H), 6.12 (d, J = 13 Hz, 1H), 6.42 (d, J = 13 Hz, 1H, 7.24 ppm (s, 1H)

Beispiel 2

Herstellung von 2-Methyl-2-acetoxy-4-chlor-3-butenal (Formel III mit $R = CH_3$ ; Hal = Cl).

a) mit wasserfreier Peressigsäure

Zu einer Lösung von 60 g 1-Acetoxy-2-methyl-4-chlor-1,3-butadien in 70 g Eisessig wurden bei 100 ± 2 °C unter Rühren innerhalb von 20 Minuten 304 g einer Eisessig-Lösung gegeben, die 12 Gew.% Peressigsäure enthielt. Man rührte weitere 20 Minuten bei dieser Temperatur nach. Unumgesetzte Peressigsäure war nicht mehr im Reaktionsgemisch nachweisbar. Man zog nun die Essigsäure am Rotationsverdampfer bei einem Druck von etwa 20 mbar weitgehend ab. Durch fraktionierte Destillation des Rückstandes erhielt man 35,5 g 2-Methyl-2-acetoxy-4-chlor-3-butenal vom Siedepunkt 59 bis 61 °C/0,5 mbar, $n_D^{20} = 1.465\,2$ (54 % d. Th., bezogen auf eingesetztes 1-Acetoxy-2-methyl-4-chlor-1,3-butadien).

$^1$H-NMR-Spektrum (Lösungsmittel $CDCl_3$, TMS als innerer Standard : $\delta$ = 1.52 (s, 3H), 2.14 (s, 3H), 6.0 (d, J = 13 Hz, 1H), 6.47 (d, J = 13 Hz, 1H), 9.35 ppm (s, 1H)

b) mit m-Chlorperbenzoesäure

Zu einer Lösung von 2,6 g 1-Acetoxy-2-methyl-4-chlor-1,3-butadien in 25 ml Chloroform wurden bei 30 ± 2 °C unter Rühren innerhalb von 20 Minuten 4,3 g m-Chlorperbenzoesäure in 40 ml Chloroform

getropft. Man rührte eine weitere Stunde bei 30 °C, dann über Nacht bei Raumtemperatur nach. Unumgesetzte Persäure war nicht mehr im Reaktionsgemisch nachweisbar. Die Chloroformphase wurde zweimal mit je 20 ml Wasser, das insgesamt 2,5 g Natriumbicarbonat enthielt, dann mit 20 ml Wasser gewaschen und über Natriumsulfat getrocknet. Das Chloroform wurde am Rotationsverdampfer abgezogen. Durch Kugelrohrdestillation des Rückstandes bei 75 bis 90 °C/0,6 mbar wurden 0,78 g 2-Methyl-2-acetoxy-4-chlor-3-butenal (27 %, bezogen auf eingesetztes 1-Acetoxy-2-methyl-4-chlor-1,3-butadien) erhalten.

c) mit Wasserstoffperoxid

Zu einem Gemisch aus 10,6 g 1-Acetoxy-2-methyl-4-chlor-1,3-butadien, 28,6 g Acetanhydrid und 60 g Eisessig wurden innerhalb von 13 Minuten bei 100 °C 6,5 g Wasserstoffperoxid (50 %) getropft. Anschließend wurde 20 Minuten bei dieser Temperatur nachgerührt. Nach dem Abziehen des Eisessig am Rotationsverdampfer (40 °C/20 mbar) wurde der Rückstand durch Kugelrohrdestillation bei 75 bis 120 °C/0,6 mbar gereinigt. Hierbei wurden 6,7 g 2-Methyl-2-acetoxy-4-chlor-3-butenal (57 %, bezogen auf eingesetztes 1-Acetoxy-2-methyl-4-chlor-1,3-butadien) erhalten.

Beispiel 3

Gemeinsame Herstellung von 2-Methyl-2-acetoxy-4-chlor-3-butenal und 2-Methyl-4,4-diacetoxy-2-butenal

Ein Gemisch aus 82 g 1-Acetoxy-2-methyl-4-chlor-1,3-butadien und 67 g 2-Methyl-1,4-diacetoxy-1,3-butadien, das nach der im Beispiel 1a beschriebenen Umsetzung, Aufarbeitung und Destillation erhalten worden war, wurde in 400 g Eisessig gelöst und bei 90 °C innerhalb von einer Stunde mit 800 g 12,4 Gew.%iger Peressigsäure versetzt. Nachdem das Reaktionsgemisch 16 h bei Raumtemperatur gestanden hatte, wurde die Essigsäure am Rotationsverdampter bei einem Druck von etwa 20 mbar abgezogen. Durch fraktionierte Destillation des Rückstandes wurden 44 g 2-Methyl-2-acetoxy-4-chlor-3-butenal vom Siedepunkt 62 bis 65 °C/1,6 mbar und 25 g 2-Methyl-4,4-diacetoxy-2-butenal vom Siedepunkt 93 bis 97 °C/1,6 mbar erhalten (49 bzw. 34 %, bezogen auf eingesetztes 1-Acetoxy-2-methyl-4-chlor-1,3-butadien bzw. 2-Methyl-1,4-diacetoxy-1,3-butadien).

Beispiel 4

Herstellung von 2,6-Dimethyl-6-acetoxy-8-chlor-2,4,7-octatriensäureethylester (Verwendung von III für die Herstellung IV ; $R^3 = CH_3$, $R^4 = COOC_2H_5$)

Zu 500 g Butylenoxid-1,2 wurden unter Rühren bei 30 °C 424,5 g (3-Ethoxycarbonyl-3-methyl-2-propenyl)-triphenyl-phosphoniumchlorid und dan allmählich 176,5 g 2-Methyl-2-acetoxy-4-chlor-3-butenal gegeben. Die Reaktionsmischung erwärmte sich während des Zutropfens des Butenals auf ca. 50 °C. Nachdem sich die Reaktionsmischung wieder abgekühlt hatte, wurde noch 24 h bei Raumtemperatur nachgerührt. Dann wurde im Rotationsverdampfer bei ≤ 40 °C auf das halbe Volumen eingeengt und bei —20 °C die Hauptmenge (192 g) des entstandenen Triphenylphosphinoxids auskristallisiert und abgesaugt. Die Mutterlauge (484,5 g), die den 2,6-Dimethyl-6-acetoxy-8-chlor-2,4,7-octatriensäureethylester enthielt, ließ sich für weitere Umsetzungen dieser Verbindung einsetzen. Für die Gewinnung der reinen Verbindung IV wurden 5 g der Mutterlauge über 200 g Kieselgel chromatographiert (Eluens : n-Hexan/Aceton 5 : 3) ; die vereinigten, die Verbindung IV enthaltenden Fraktionen ergaben nach dem Einengen 2,2 g 2,6-Dimethyl-6-acetoxy-8-chlor-2,4,7-octatriensäureethylester als Isomerengemisch (55 % all-E, 45 % 2E, 4Z, 7E). Die Ausbeute betrug demnach 74,4 %.

$^1$H-NMR-Spektrum (Lösungsmittel $CDCl_3$, TMS als innerer Standard) : all-E-Isomeres : δ = 1,3 (T, 3H), 1,68 (S, 3H), 1,96 (S, 3H), 2,04 (S, 3H), 4,22 (Q, 2H), 6,21 (D, 1H), 6,22 (D, 1H), 6,30 (D, 1H), 6,51 (DD, 1H), 7,18 (D, 1H).

(2E, 4Z, 7E)-Isomeres : δ = 1,31 (T, 3H), 1,71 (S, 3H), 1,94 (S, 3H), 2,05 (S, 3H), 4,23 (Q, 2H), 5,89 (D, 1H), 6,29 (D, 1H), 6,34 (T, 1H), 6,37 (D, 1H), 7,64 (D, 1H).

**Patentansprüche**

1. Halogen enthaltende Acyloxy-methyl-butene der Formel

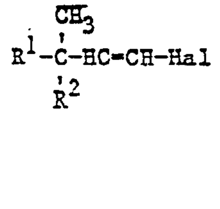

(I)

in der $R^1$ den

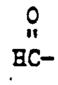

Rest oder zusammen mit $R^2$ einen

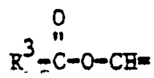

Rest $R^2$ einen

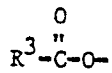

Rest oder zusamen mit $R^1$ einen

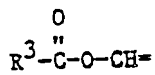

Rest Hal ein Chlor- oder Bromatom und $R^3$ ein Wasserstoffatom oder einen Alkylrest mit 1 bis 16 C-Atomen bedeuten.

2. 1-Acyloxy-2-methyl-4-halogen-1,3-butadiene der Formel

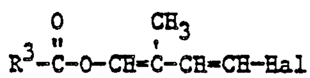

(II)

in der Hal und $R^3$ die in Anspruch 1 genannte Bedeutung haben.

3. 2-Methyl-2-acyloxy-4-halogen-3-butenale der Formel

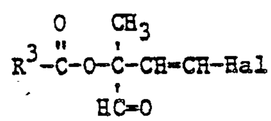

(III)

in der $R^3$ und Hal die in Anspruch 1 genannte Bedeutung haben.

4. Verfahren zur Herstellung von 2-Methyl-2-acyloxy-4-halogen-3-butenalen nach Anspruch 3, dadurch gekennzeichnet, daß man 1-Acyloxy-2-methyl-4-halogen-1,3-butadiene nach Anspruch 2 mit Sauerstoff oder Sauerstoff abgebenden Mitteln umsetzt.

5. Verwendung von 2-Methyl-2-acyloxy-4-halogen-3-butenalen nach Anspruch 3 für die Herstellung von Verbindungen der Formel

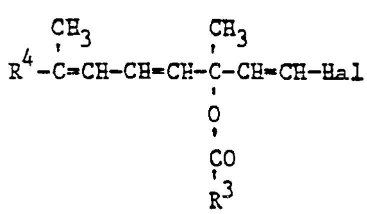

(IV)

in der Hal und $R^3$ die in Anspruch 3 genannten Bedeutungen haben und $R^4$ für einen $R^5$OOC-Rest, eine Formylgruppe oder eine acetalisierte Formylgruppe steht, wobei $R^5$ einen niedermolekularen Alkylrest bedeutet.

**Claims**

1. A halogen-containing acyloxymethylbutene of the formula

(See formula (I) page 7)

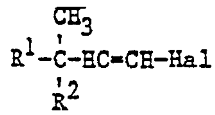

(I)

where $R^1$ is an

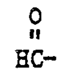

radical or, together with $R^2$, an

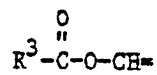

radical, $R^2$ is an

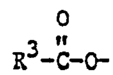

radical or, together with $R^1$, an

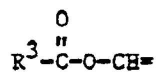

radical, Hal is chlorine or bromine, and $R^3$ is hydrogen or alkyl of 1 to 16 carbon atoms.

2. A 1-acyloxy-2-methyl-4-halo-1,3-butadiene of the formula

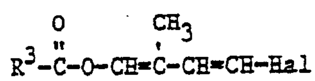

(II)

where Hal and $R^3$ have the meanings given in claim 1.

3. A 2-methyl-2-acyloxy-4-halo-3-butenal of the formula

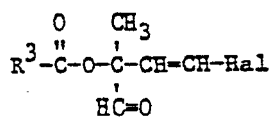

(III)

where $R^3$ and Hal have the meanings given in claim 1.

4. A process for the preparation of a 2-methyl-2-acyloxy-4-halo-3-butenal as claimed in claim 3, wherein a 1-acyloxy-2-methyl-4-halo-1,3-butadiene as claimed in claim 2 is reacted with oxygen or an oxygen-donating agent.

5. The use of a 2-methyl-2-acyloxy-4-halo-3-butenal as claimed in claim 3 for the preparation of a compound of the formula

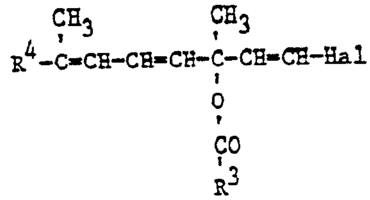

(IV)

where Hal and $R^3$ have the meanings given in claim 3, and $R^4$ is an $R^5OOC$ radical, a formyl group or an acetalized formyl group, $R^5$ denoting a low-molecular weight alkyl radical.

**Revendications**

1. Acyloxy-méthyl-butène contenant un halogène, répondant à la formule

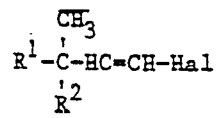

(I)

dans laquelle $R^1$ est un radical

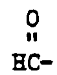

ou conjointement avec $R^2$ un radical

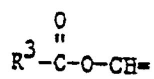

$R^2$ un radical

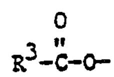

ou conjointement avec $R^1$

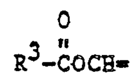

Hal un atome de chlore ou de brome, et $R^3$ un atome d'hydrogène ou un radical alkyle en $C_1$-$C_{16}$.

2. 1-Acyloxy-2-méthyl-4-halogéno-1,3-butadiène de formule

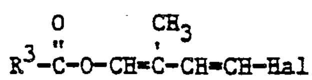

(II)

dans laquelle Hal et $R^3$ sont tels que définis dans la revendication 1.

3. 2-Méthyl-2-acyloxy-4-halogéno-3-buténals de formule

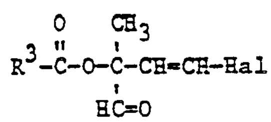

(III)

dans laquelle $R^3$ et Hal sont tels que définis dans la revendication 1.

4. Procédé pour la préparation de 2-méthyl-2-acyloxy-4-halogéno-3-buténals selon la revendication 3, caractérisé en ce que l'on fait réagir du 1-acyloxy-2-méthyl-4-halogéno-1,3-butadiène selon la revendication 2 avec de l'oxygène ou un agent dégageant de l'oxygène.

5. Utilisation des 2-méthyl-2-acyloxy-4-halogéno-3-buténals selon la revendication 3 pour la préparation des composés de formule

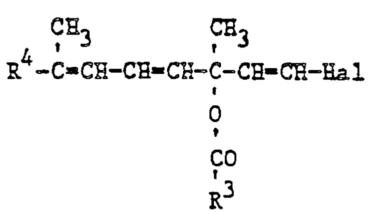

(IV)

dans laquelle Hal et $R^3$ sont tels que définis dans la revendication 3 et $R^4$ un radical $R^5OOC$—, un groupe formyle ou un groupe formyle acétylé, $R^5$ étant un radical alkyle à bas poids moléculaire.